# EUROPEAN PATENT APPLICATION

(11) **EP 4 636 074 A1**
(43) Date of publication of application: **22.10.2025**
(21) Application number: 23903709.6
(22) Date of filing: 16.10.2023
(51) Int. Cl.: C12N 5/00

(54) **METHOD FOR ORGANOID SEED PRODUCTION, AND ORGANOID SEED PRODUCTION APPARATUS FOR SAME**

(30) Priority: 14.12.2022 KR 20220174402; 12.10.2023 KR 20230135651
(71) Applicant: Radahaim Co., Ltd., Seongnam-si, Gyeonggi-do 13466 (KR)
(72) Inventor: LEE, Heon Ju, Yongin-si Gyeonggi-do 17009 (KR); AN, Jee Young, Seoul 03066 (KR); LIM, Hyun Wook, Seoul 04726 (KR)
(74) Representative: Hautier IP - MC/EP
(86) International application number: PCT/KR2023/015958
(87) International publication number: WO 2024/128505

(57) **Abstract**

The present disclosure provides an organoid seed production method and apparatus for organoid production, the method comprising a step of forming organoid seeds in a microfluidic chip by means of an organoid seed composition, the organoid seeds being suspended in a droplet state in a solution.

## Description

### [Technical Field]

The present disclosure relates to an organoid production method, and more particularly, to an organoid production method and apparatus, including a method and apparatus for producing organoid seeds used for producing organoids.

### [Background Art]

In a process of developing new drugs, animal experiments on animals similar to humans are required prior to conducting clinical trials. Animals such as rats and rabbits are used in animal experiments, and the ethics therefor are questionable. In addition, since animal experimental models have different genetic or biological characteristics from humans, drug tests using existing animal experimental models have limitations in the reliability of drug test reactions, such as side effects that have not been found in animal experiments, but are found in humans.

Moreover, recently, as the US FDA removed the mandatory provision of requiring animal experiments as a condition for new drug approval, various methods for new preclinical test models for new drug development have been studied.

However, conventional 2D cell line culture methods have the disadvantage of being difficult to reproduce the unique properties and characteristics of tissues, and cancer patient-derived xenograft models are also used as an alternative, but have the disadvantage of being unsuitable for large-scale drug screening.

Therefore, organoids have recently attracted significant attention as a new preclinical test model for new drug development.

The organoids are organoids produced by culturing or recombining stem cells in a three-dimensional manner, and are derived from various organs in the body and contain stem cells therein to form aggregates that may differentiate into cell bodies. In addition, organoids derived from each organ in the body have a structure very similar to the corresponding organ, and thus may be used as models capable of replacing cell and animal experiments.

However, conventionally cultured organoids had limitations in use in actual new drug development due to problems with consistency and reproducibility of study results. This is because cell culture varies depending on a laboratory environment, and the size and degree of differentiation vary depending on a batch even when cultured under the same conditions, so that there are limitations to obtain statistically significant data.

To solve the problems in the related art, organoid production technology is required so that cultured organoids may be produced in a homogeneous form under certain conditions even while enabling mass production of organoids.

### [Disclosure]

### [Technical Problem]

An object of the present disclosure is to provide an organoid seed production apparatus and method for organoid production to culture organoids by continuously producing separately organoid seeds in a droplet form in a microchannel.

Another object of the present disclosure is to provide an organoid seed production method and apparatus, which is advantageous for three-dimensional culture of organoid seeds by producing the organoid seeds in a suspended state in a solution.

### [Technical Solution]

According to one aspect of the present disclosure, there is provided an organoid seed production method for organoid production, including a step of forming organoid seeds suspended in a droplet form in a solution using an organoid seed composition in a microfluidic chip; and a step of collecting the organoid seeds.

According to one aspect of the present disclosure, the organoid seed production method further includes a step of gelling the organoid seeds.

According to one aspect of the present disclosure, the organoid seed composition may include stem cells, growth factors, biodegradable materials, and an extracellular matrix.

According to one aspect of the present disclosure, the organoid seed composition includes magnetic nanoparticles.

According to one aspect of the present disclosure, the microfluidic chip includes a plate having a microchannel through which organoid seeds move, and the plate includes an insertion hole for inserting a syringe tip into the microchannel, a syringe tip extension region forming a front portion of the microchannel and through which the syringe tip extends, a first liquid port for injecting a solution into a first bonding part formed in the syringe tip extension region, and an outlet for collecting organoid seeds that move along the microchannel in a suspended state in the solution.

According to one aspect of the present disclosure, in step a), the organoid seed composition is injected into the microchannel at a first flow velocity V1 through the syringe tip, and the solution is injected between the inner surface of the microchannel and the outer surface of the syringe tip at V2, which form a relationship of V1 < V2.

According to one aspect of the present disclosure, a surface tension N1 of the organoid seed composition and a surface tension N2 of the solution form a relationship of N1 > N2.

According to one aspect of the present disclosure, the microfluidic chip includes a second bonding part formed on a path of the microchannel along which the organoid seeds move while being suspended in the solution, and a second liquid port for injecting a second solution into the second bonding part, and step b) is performed by injecting a gelation solution capable of gelling the organoid seeds into the second liquid port by reacting with the biodegradable material contained in the organoid seed composition.

According to one aspect of the present disclosure, the organoid seed composition includes a component that is gelled by UV as the biodegradable material, and step b) is performed by irradiating UV to the organoid seeds that move along the microchannel while being suspended in the solution.

According to another aspect of the present disclosure, there is provided an organoid seed production apparatus for producing organoids using an organoid seed composition, including: a microfluidic chip formed of a plate including a microchannel; an insertion hole for inserting a syringe tip into the microchannel; a syringe tip extension region forming a part of the microchannel and through which the syringe tip extends; a first liquid port for injecting a solution into a first bonding part formed in the syringe tip region, and an outlet for collecting the organoid seeds that move along the microchannel in a suspended state in the solution; a first syringe having the syringe tip at an end and containing the organoid seed composition therein and injecting the organoid seed composition into the microchannel through the syringe tip; a second syringe connected to the first liquid port and containing the solution and injecting the solution into the microchannel through the first bonding part; a first actuator connected to the first syringe to inject the organoid seed composition into the microchannel; and a second actuator connected to the second syringe to inject the solution into the microchannel.

According to another aspect of the present disclosure, the microfluidic chip further includes a second bonding part formed on a path of the microchannel along which the organoid seeds move while being suspended in the solution, and a second liquid port for injecting a second solution into the second bonding part, and as the second solution, a gelation solution capable of gelling the organoid seeds is injected by reacting with the biodegradable material contained in the organoid seed composition.

According to another aspect of the present disclosure, the organoid seed composition includes a biodegradable material as a component so as to gel the organoid seeds in response to UV, and includes a UV irradiation device that irradiates UV to the organoid seeds in a droplet state which moves along the microchannel.

According to another aspect of the present disclosure, the plate of the microfluidic chip includes a shielded seed formation region covered with a shielding cover and a UV irradiation region exposed to the UV irradiation device, so that UV is irradiated to the organoid seeds moving along the microchannel in the UV irradiation region.

According to another aspect of the present disclosure, in the microfluidic chip, an upper plate and a lower plate are bonded to each other to form the plate, and the lower plate includes a first lower plate corresponding to the shielded seed formation region and a second lower plate corresponding to the UV irradiation region, in which the first lower plate is formed of a UV transparent material and the second lower plate is formed of a UV reflective material.

### [Advantageous Effects]

According to the present disclosure, it is possible to continuously produce separately organoid seeds which move in a microchannel while being suspended in a solution.

According to the present disclosure, the organoid seeds can be gelled by biodegradable materials included in the organoid seed composition. Therefore, it is possible to maintain a spherical shape while evenly distributing stem cells, growth factors, extracellular matrix, and magnetic nanoparticles in the organoid seeds, and to create an environment in which cells can grow, agglomerate together, and differentiate into different tissues.

The organoid seeds produced according to the present disclosure can be cultured into organoids by transferring the organoid seeds to a culture device and exchanging a culture medium. It is possible to produce and then culture organoid seeds with uniform shape and size and to continuously produce organoid seeds separately in the form of containing magnetic nanoparticles. Therefore, it is advantageous for three-dimensional suspension culture of organoid seeds. In addition, after three-dimensional culture, the organoids can be sorted by size using an organoid sorter and provided for experiments such as new drug development and the like.

### [Description of Drawings]

FIG. 1 is a flow chart of an organoid production method according to the present disclosure.
FIG. 2 is a flow chart of an organoid seed production method according to an embodiment of the present disclosure.
FIG. 3 is a plan view of an embodiment of a microfluidic chip forming an organoid seed production apparatus according to an embodiment of the present disclosure.
FIG. 4 is a side view of a microfluidic chip forming an organoid seed production apparatus according to an embodiment of the present disclosure.
FIG. 5 is a plan view of another embodiment of a microfluidic chip forming an organoid seed production apparatus according to an embodiment of the present disclosure.
FIG. 6 is a plan view of another embodiment of a microfluidic chip forming an organoid seed production apparatus according to an embodiment of the present disclosure.
FIG. 7 is a diagram illustrating a state in which a shielding cover is removed from the plan view of FIG. 6.
FIG. 8 is a side view of the microfluidic chip illustrated in FIG. 6.
FIG. 9 is a diagram showing Experimental Embodiment to confirm the formation of organoid seed droplets in a microchannel of a microfluidic chip.
FIG. 10 is a diagram showing a culture appearance of organoid seeds produced according to Experimental Embodiment of the present disclosure.

### [Best Mode]

Hereinafter, embodiments of the present disclosure will be described with reference to the accompanying drawings.

The present disclosure may have various modifications and various embodiments, and specific embodiments will be illustrated in the drawings and described in detail in the detailed description. However, it should be understood that the present disclosure is not limited to specific embodiments, but covers all the modifications, equivalents and replacements included within the idea and technical scope of the present disclosure. In describing the present disclosure, a detailed description of known related arts will be omitted if it is determined that they make the gist of the present disclosure unclear.

In this specification, the "organoid seed composition" means a cell composition including stem cells that are formed in a spherical shape for producing organoid seeds, and the organoid seed means a form that may be cultured in a culture medium using the organoid seed composition to become an organoid.

In addition, the term "organoid seeds" used in the present disclosure include both organoid seeds in a droplet state before gelation, i.e., organoid seed droplets, and gelated organoid seed gels, which are referred to separately only when a distinction is required.

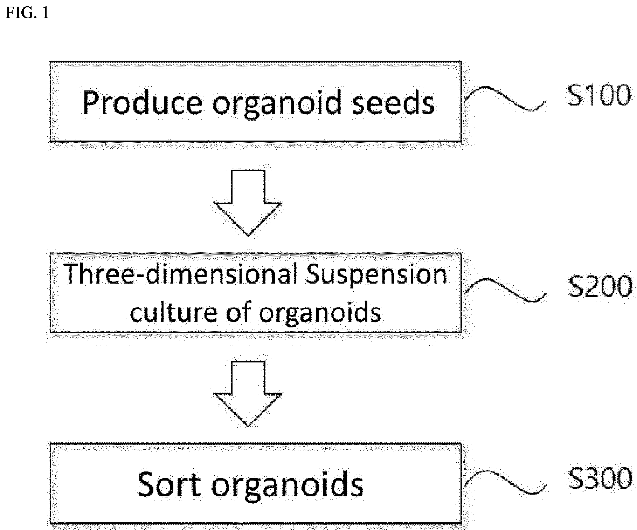

FIG. 1 is a flow chart of an organoid production method according to the present disclosure. Referring to FIG. 1, an organoid three-dimensional culture method includes an organoid seed production step (S100), an organoid three-dimensional suspension culture step (S200), and an organoid sorting step (S300).

The organoid seed production step is a step of producing organoid seeds in a suspended form in a solution (liquid medium) in a microchannel.

The organoid seeds refer to organoid seeds in which an organoid seed composition is formed in a spherical shape in the solution, and the organoid seeds are produced in the form of droplets in a microfluidic chip using the organoid seed composition, and then gelled. The organoid seeds are transferred to the organoid three-dimensional suspension culture apparatus.

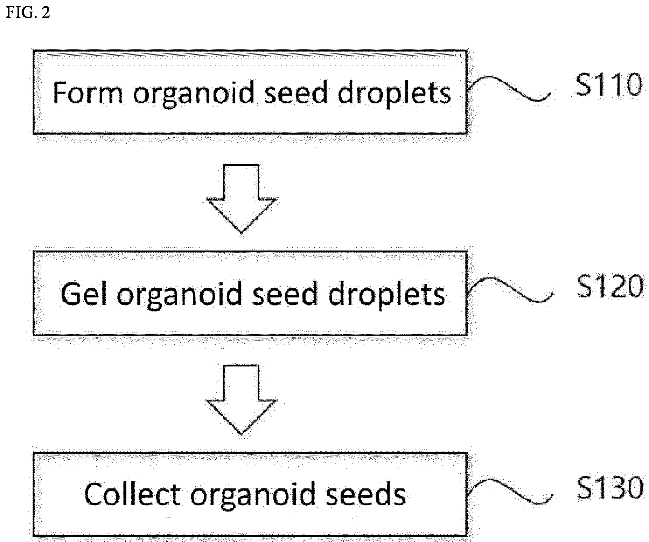

FIG. 2 is a diagram illustrating an organoid seed production method according to an embodiment of the present disclosure.

In the organoid seed production method according to an embodiment of the present disclosure, a microfluidic chip having a microchannel is used. The organoid seed production method includes supplying an organoid seed composition into a microchannel to continuously form organoid seed droplets in a form of being suspended and moved in the solution (S110); and gelling the organoid seed droplets (S120). In addition, the method includes a step of collecting organoid seeds (S130).

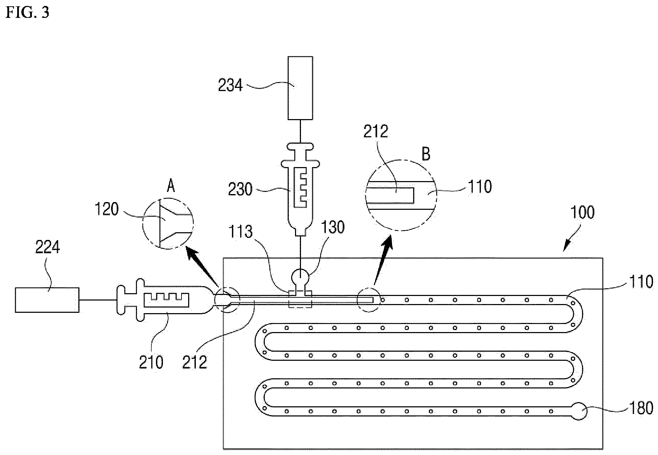

The gelation of organoid seeds may be performed continuously after formation of organoid seed droplets in the microfluidic chip, as illustrated in FIG. 2. However, it is not limited thereto, and as expected from FIG. 3, the organoid seeds may be produced in the form of droplets, and then collected from the microfluidic chip and gelled in a separate apparatus.

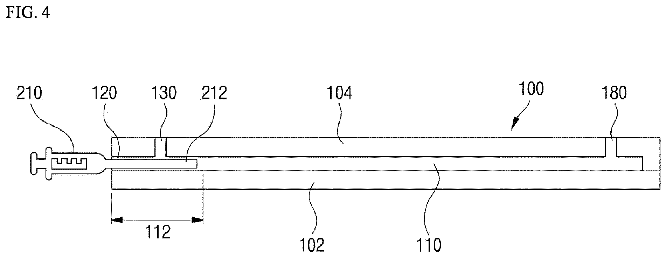

FIG. 3 is a plan view of an embodiment of a microfluidic chip forming an organoid seed production apparatus according to an embodiment of the present disclosure, and FIG. 4 is a side view of the microfluidic chip.

A microfluidic chip 100 is manufactured as a plate formed by bonding a lower plate 102 and an upper plate 104, and includes a microchannel 110 therein.

According to the embodiment of the present disclosure, the inner diameter of the microchannel may be 100 µm to 5 mm. The inner diameter of the microchannel may be preferably 1 mm or less, and more preferably 500 µm to 1 mm.

In the microfluidic chip 100, the microchannel 110 may be extended in a zigzag arrangement to extend the length in the microfluidic chip 100.

The lower plate 102 and the upper plate 104 of the microfluidic chip 100 may be manufactured from inorganic materials such as glass or silicon, and may be manufactured using polymer materials such as poly(dimethyl) siloxane (PDMS) or polymethylmethacrylate (PMMA) to be bonded to each other.

The lower plate 102 and the upper plate 104 may be formed of different materials. For example, the lower plate 102 may be formed of a glass material, the upper plate 104 may be formed of a PDMS material, and the microchannel may be formed through a soft lithography process, etc.

The microfluidic chip 100 includes an insertion hole 120, a first liquid port 130, and an outlet 180, which are connected with the microchannel 110 extending from the inside. In addition, the microfluidic chip 100 includes a first bonding part 113 which is a part of the microchannel 110 and forms a connection point with the first liquid port 130.

In this specification, with respect to an extension direction of the microchannel 110, the insertion hole 120 side is referred to as a front side, and the outlet 180 side is referred to as a rear side.

The insertion hole 120 is a starting point of the microchannel 110 and forms an inlet into which a syringe tip 212 is inserted. A syringe tip section 112 extending in a straight line is formed in the front part of the microchannel 110 extending from the insertion hole 120. The syringe tip section 112 forms a path through which the syringe tip 212 is inserted and extended as a part of the microchannel 110.

A first syringe 210 is coupled to the insertion hole 120. Referring to an enlarged part A of FIG. 3, the front end of the insertion hole 120 which comes into contact with the first syringe 210 is processed into a cone shape. Therefore, sealing is easy after the syringe tip 212 is inserted.

The first syringe 210 contains an organoid seed composition. The first syringe 210 is a chamber that contains the organoid seed composition.

According to the embodiment of the present disclosure, the organoid seed composition includes stem cells, biodegradable materials, and growth factors. In addition, the organoid seed composition may further include an extracellular matrix.

Stem cells are precursor cells for organoid differentiation and may include adult stem cells (ASCs), embryonic stem cells (ESCs), and/or induced pluripotent stem cells (iPSCs). For example, the stem cells may be primary stem cells, proliferated stem cells, or partially differentiated stem cells. Specifically, the stem cells may be primary cells, which may be obtained directly from living tissue. In addition, the stem cells may be secondary cells, which may be cultured and/or subcultured cells.

As the biodegradable materials, gelatin, gelatin methacrylate (GelMA), collagen, poly(ethylene glycol) (PEG), Pluronic^{®}, alginate, and the like may be used. The biodegradable materials are contained for gelation of organoid seeds, and the gelation process varies depending on a type of biodegradable material.

The biodegradable materials serve to maintain the shape of organoid seeds through gelation and may be used as components that construct tissues as cells grow. The biodegradable materials are gelled into a hydrogel state containing stem cells, growth factors, and extracellular matrix therein. The gelled biodegradable materials allow the exchange of oxygen and carbon dioxide and the absorption of nutrients supplied from the medium therein, and retain moisture. The biodegradable materials are degraded and then disappear as the stem cells grow and are cultured into organoids.

The extracellular matrix (ECM) may be a component included to differentiate the stem cells into organoids. The stem cells may differentiate into organoids by coming into contact with the extracellular matrix, and a type of extracellular matrix and the like may be controlled according to the characteristics of a desired organoid.

According to the embodiment of the present disclosure, the extracellular matrix may be obtained by culturing extracellular matrix-producing cells, such as epithelial cells, endothelial cells, exterior endoderm-like cells, or fibroblasts, and then removing the cells. The extracellular matrix-producing cells may be selected from chondrocytes which mainly produce collagen and proteoglycans; fibroblasts which mainly produce type IV collagen, laminin, interstitial procollagen and fibronectin; and colonic myofibroblasts which mainly produce collagen (types I, III and V), chondroitin sulfate proteoglycan, hyaluronic acid, fibronectin and tenascin-C. In addition, the extracellular matrix may be applied with a commercially available extracellular matrix such as Matrigel.

The growth factors are biologically active substances of the protein family that exist in the body and regulate cell growth, proliferation, and differentiation. As the growth factors, tens of types such as EGF, FGF, IGF, KGF, VEGF, and PDGF are known.

According to the embodiment of the present disclosure, the organoid seed composition may include magnetic nanoparticles.

The magnetic nanoparticles included in the organoid seeds enable organoid culture by suspending the magnetic nanoparticles without contacting the surface of the channel in which the organoid seeds are positioned, under an environment where magnetic force is applied.

The magnetic nanoparticles may exhibit cytotoxicity, and are known to depend on a size and a concentration. According to an embodiment of the present disclosure, the magnetic nanoparticles are used at a diameter of 50 nm or less and a concentration of 50 µg/ml or less, and in this case, it was determined that there was no cytotoxicity. The magnetic nanoparticles are not limited thereto, and may be used at any size and concentration without cytotoxicity.

The magnetic nanoparticles may be included separately or in multiples in the organoid seeds.

The first syringe 210 is connected to a first actuator 224 so that the organoid seed composition may be provided into the microchannel 110 through the syringe tip 212 at predetermined flow velocity and amount.

The outer diameter of the syringe tip 212 for forming the organoid seed droplets is formed to correspond to the inner diameter of the microchannel, and according to the embodiment of the present disclosure, the outer diameter is preferably 100 µm to 900 µm. According to experiments of the present researchers, the formation of organoid seed droplets depends on the size of the outer diameter of the syringe tip 212 and is irrelevant of the size of the inner diameter.

The first actuator 224 may be a syringe pump using a step motor, a pneumatic actuator using air pressure, or the like.

The first syringe 210 is coupled with the microchannel 110 through the insertion hole 120 while being coupled with the syringe tip 212. The syringe tip 212 extends through the syringe tip section 112 of the microchannel 110.

The syringe tip 212 injects the organoid seed composition into the microchannel 110. As shown in an enlarged portion B of FIG. 3, a blunt-shaped end of the syringe tip 212 is used. The cross-sectional shape may have a circular or a vertically sharp processed shape.

The outer diameter of the syringe tip 212 determines the diameter of the spherical organoid seed droplet. Therefore, the syringe tip 212 may be replaced according to an intended size of the organoid seed droplet.

The syringe tip 212 has a length that allows the organoid seed composition solution introduced into the microchannel 110 through the syringe tip 212 and the solution introduced through the first liquid port 130 to maintain a laminar flow.

A first bonding part 113, which is a bonding part between the first liquid port 130 and the microchannel 110, is formed in the syringe tip section 112. The first liquid port 130 is formed in the microfluidic chip 100 to inject the solution into the first bonding part 113. The first bonding part 113 is a point where the solution introduced into the first liquid port 130 joins the microchannel 110.

Since the first bonding part 113 is formed in the syringe tip section 112 from which the syringe tip 212 extends, the solution introduced through the first liquid port 130 is transferred backward between the outer surface of the syringe tip 212 and the outer surface of the microchannel 110. The organoid seed composition and the solution flow laminarly in a form similar to a coaxial double conduit with the syringe tip 212 as a boundary, respectively.

The solution introduced through the first liquid port 130 is a medium involved in the formation of organoid seed droplets and the transfer of organoid seed droplets, and may be a medium or a saline solution.

The first liquid port 130 is connected with a second syringe 230 containing the solution, and connected with a second actuator 234 to control the flow velocity of the solution provided to the first liquid port 130.

The solution introduced into the first liquid port 130 is involved in the dropletization of the organoid seed composition discharged from the syringe tip 212 into the microchannel 110. The produced organoid seed droplets are allowed to move in a line along the microchannel 110 while suspended in the solution.

According to the embodiment of the present disclosure, the organoid seed composition is formed into droplets and moves along the microchannel due to the flow rate and flow velocity of the organoid seed composition introduced through the syringe tip 212 and the solution introduced through the first liquid port 130, and a difference in surface tension between the organoid seed composition and the solution.

When the flow velocity of the organoid seed composition injected from the syringe tip 212 into the microchannel 110 is V1, the flow velocity of the solution supplied through the first liquid port 130 and moving between the outer surface of the syringe tip 212 and the inner surface of the microchannel 110 via the first bonding part 113 is V2, the surface tension of the organoid seed composition discharged from the syringe tip 212 is N1, and the surface tension of the solution injected through the first liquid port 130 is N2, the organoid seed composition and the solution form a laminar flow, and a relationship of V1 < V2 and N1 > N2 is established.

Since the inner and outer diameters of the syringe tip 212 and the inner diameter of the microchannel 110 are determined, the flow velocity is controlled to establish a relationship V1 < V2 by using a flow rate Q1 injected into the syringe tip 212 through the first actuator and Q2 supplied between the outer surface of the syringe tip 212 and the inner surface of the microchannel 110 through the second actuator.

Through this, the organoid seed composition is produced at a constant speed while forming spherical droplets suspended in the solution and moves toward the outlet 180 along the microchannel 110.

According to the embodiment of the present disclosure, the organoid seed droplets produced in the microchannel 110 are gelled. To this end, the microfluidic chip 100 may include a second liquid port 140 or a UV irradiation region 155.

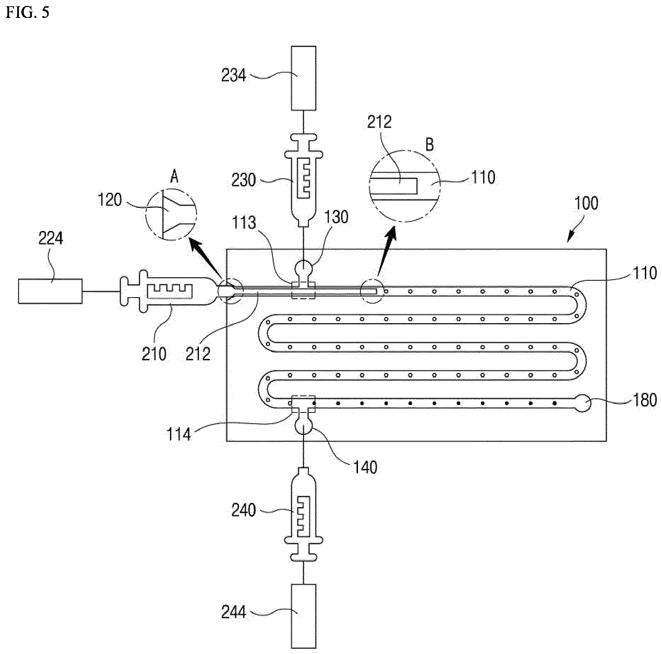

FIG. 5 shows an embodiment in which a first liquid port for injecting a gelation solution is integrated with a microfluidic chip to induce gelation of organoid seed droplets in the microfluidic chip forming an organoid seed production apparatus according to an embodiment of the present disclosure.

Referring to FIG. 5, the microfluidic chip 100 includes a second liquid port 140 for injecting a second solution for gelation of the organoid seed droplets.

The gelation solution injected through the second liquid port 140 joins the microchannel 110 through the second bonding part 114.

The second liquid port 140 is connected with a third syringe 240 containing the second solution, and connected with a third actuator 244 to control the flow velocity of the liquid provided to the second liquid port 140.

The second solution is a gelation solution that induces gelation of the organoid seed droplets, and reacts with the biodegradable material contained in the organoid seed composition to gel the organoid seeds. The gelation solution provided through the second liquid port 140 may vary depending on a biodegradable material contained in the organoid seed composition.

In addition, the organoid seed composition injected using the first syringe for gelation of organoid seeds, the solution injected using the second syringe, and the gelation solution injected using the third syringe may be controlled to an appropriate temperature for smooth performance of the gelation reaction.

For example, when the biodegradable material included in the organoid seed composition includes alginate, the gelation solution may include calcium chloride (CaCl₂).

In addition, for example, when the biodegradable materials included in the organoid seed composition are collagen and gelatin, the gelation solution may be a temperature-controlled medium or saline solution. The organoid seed composition including collagen and gelatin is injected through the syringe tip 212 at a temperature of 37°C, a medium or saline solution is injected at a temperature of 37°C through the first liquid port 130, and the same medium or saline solution as that injected through the first liquid port 130 may be supplied through the second liquid port 140 while maintaining the temperature at 30°C or lower.

In addition, for example, when the biodegradable material included in the organoid seed composition is pluronic, the organoid seed composition including pluronic is injected through the syringe tip 212 at a temperature of 30°C or lower, and the medium or saline solution injected through the second liquid port 140 is supplied at a temperature of 37°C to induce gelation. At this time, the medium or saline solution which is a solution injected through the first liquid port 130 is maintained at 30°C or lower.

In addition, for example, when the biodegradable material included in the organoid seed composition is fibrin, the gelation solution may be thrombin, and when the biodegradable material included in the organoid seed composition is thrombin, the gelation solution may be used with fibrin. The fibrin and thrombin react with each other to form fibrinogen drops.

In general, the organoid seed composition, the solution, and the gelation solution are supplied at a temperature of 37°C or lower above room temperature, and for a gelling reaction using a biodegradable material, the temperature of the solution injected into the syringe tip, the first liquid port, and the second liquid port may be controlled differently.

According to another embodiment of the present disclosure, the microfluidic chip 100 may be manufactured in a form in which the UV irradiation region 155 is integrated.

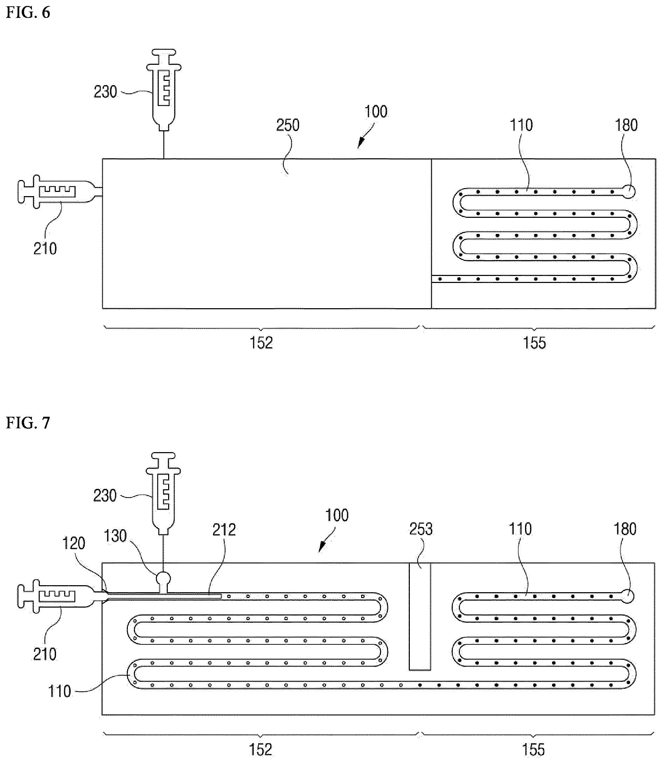

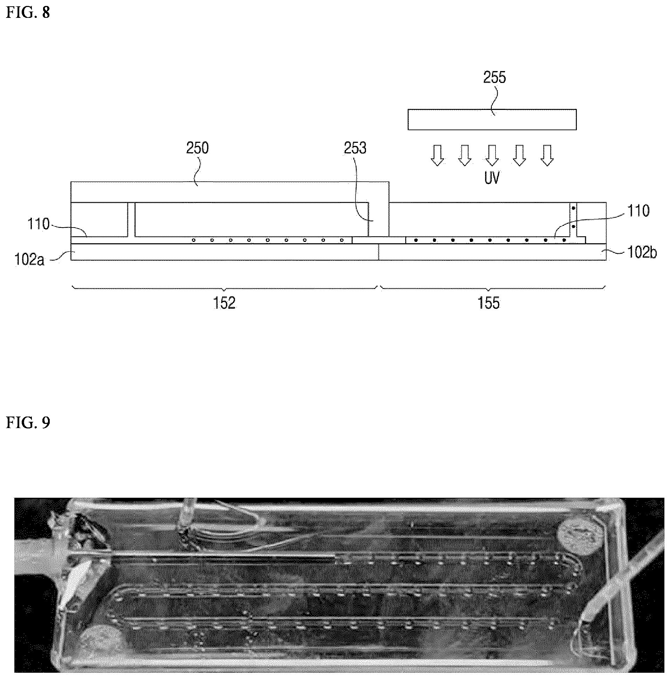

FIG. 6 is a plan view of another embodiment of a microfluidic chip forming an organoid seed production apparatus according to an embodiment of the present disclosure, FIG. 7 is a diagram illustrating a state in which a shielding cover is removed from the plan view of FIG. 6, and FIG. 8 is a side view of the microfluidic chip illustrated in FIG. 6.

Referring to FIGS. 6 to 8, the microfluidic chip 100 is divided into a seed formation region 152 covered with a shielding cover 250 and a UV irradiation region 155 exposed to a UV irradiation device 255.

The seed formation region 152 is a region where the organoid seed composition is injected to form organoid seed droplets, and includes a shielding cover 250 to be protected from UV irradiation from the UV irradiation device 255. When the seed formation region 152 may be protected from UV by means other than the shielding cover 250, such as spatial isolation, layout design of the UV irradiation device 255, etc., the shielding cover 250 may not be included.

The seed formation region 152 includes the insertion hole 120, and the first liquid port 130, and includes a region in which organoid seed droplets move along the microchannel 110. When the seed formation region 152 is protected by the shielding cover 250, the seed formation region is referred to as a shielded seed formation region.

The UV irradiation region 155 is a region where the organoid seed droplets moving along the microchannel 110 are gelled by UV.

The lower plate 102 of the microfluidic chip 100 may have the shielded seed formation region 152 and the UV irradiation region 155 formed differently from each other.

According to the embodiment of the present disclosure, a first lower plate 102a corresponding to the shielded seed formation region 152 is made of a glass material that may transmit UV, and a second lower plate 102b corresponding to the UV irradiation region 155 is formed of an acrylic material that may reflect ultraviolet rays.

The shielding cover 250 is formed of an aluminum material. The shielding cover 250 further includes a partition wall 253 that isolates the shielded seed formation region 152 and the UV irradiation region 155 in the microfluidic chip 100, and prevents UV provided to the UV irradiation region 155 from affecting the organoid seed droplets in the shielded seed formation region 152.

As described above, the microfluidic chip 100 is divided into the shielded seed formation region 152 and the UV irradiation region 155, and whether to gel the organoid seed droplets by UV or by the gelation solution injected through the second liquid port 140 may be selective.

For example, when the organoid seed composition includes alginate as a biodegradable material, the organoid seed droplets are gelled by the calcium chloride solution introduced through the second liquid port 140. Therefore, it is unnecessary to divide the microfluidic chip 100 into the shielded seed formation region 152 and the UV irradiation region 155.

The microfluidic chip 100 is manufactured in a form having both the second liquid port 140 and the UV irradiation region 155, and a suitable gelling process may be selectively performed depending on a type of biodegradable material.

When the organoid seed droplets contain gelma as a biodegradable material, the organoid seed droplets are gelated by UV irradiation.

The outlet 180 is formed at the end of the microchannel 110, and the gelled organoid seeds are collected.

The produced organoid seeds may be transferred to the organoid three-dimensional suspension culture apparatus.

Since the organoid seeds are produced into uniform size and shape by the organoid seed production apparatus, it is advantageous to be cultured into organoids having a uniform size, and since the organoid seeds are produced continuously while being spaced apart from each other, the organoid seeds may be continuously supplied separately to a subsequent process, that is, the organoid three-dimensional suspension culture apparatus.

FIG. 9 is Experiment Example performed to confirm a formation process of organoid seed droplets in a microchannel of a microfluidic chip, and it was confirmed that spherical organoid seed droplets were continuously formed with a uniform size along the microchannel.

### Experimental Embodiment

Organoid seeds were prepared using a microfluidic chip. In the microfluidic chip, a microchannel was formed as a square channel with a cross-sectional area of 1 × 1 mm², and a syringe tip with an outer diameter of 900 µm was used.

An organoid seed composition and a saline solution were injected through a first syringe and a first liquid port, and the flow rate was controlled between 0.2 ml/min and 20 ml/min. While a flow rate Q1 of the organoid seed composition injected through the syringe tip and an injection flow rate Q2 of the solution between the inner diameter of the microchannel and the outer diameter of the syringe tip were controlled, considering the inner and outer diameter dimensions of the microchannel and the syringe tip, a flow velocity V1 was controlled to be smaller than a flow velocity V2.

Specifically, the injection flow rate of the organoid seed composition was set to 25 ml/min, the injection flow rate of the solution through the first liquid port was set to 30 ml/min, and a relationship of V1 < V2 was established due to a dimensional difference between the syringe tip and the microchannel.

As an organoid seed composition, 5 × 10⁷ human embryonic kidney (HEK) cells were dispersed in 10 µl of an organoid culture medium (ThermoFisher) and stained with 10 µl of trypan blue. Thereafter, after the supernatant was removed, the organoid seed composition was prepared by adding 5 ml of alginate and 30 µg/ml of magnetic nanoparticles (MNP, particle size 20 nm).

The organoid seed composition prepared above was injected at 25 ml/min through a syringe tip, and a phosphate buffered saline (PBS) solution was injected through the first liquid port. The surface tension of the organoid seed composition was greater than the surface tension of the saline solution used as the solution.

Thereafter, a calcium chloride solution was injected through the second liquid port to gel the organoid seeds. The organoid seed composition, the saline solution, and the calcium chloride solution were injected at 30°C.

Organoid seed droplets were formed at a speed of 10 Hz using a syringe tip with an outer diameter of 900 µm, and the average diameter of 50 organoid seeds was measured to be 950 µm and the standard deviation was measured to be less than 10 µm.

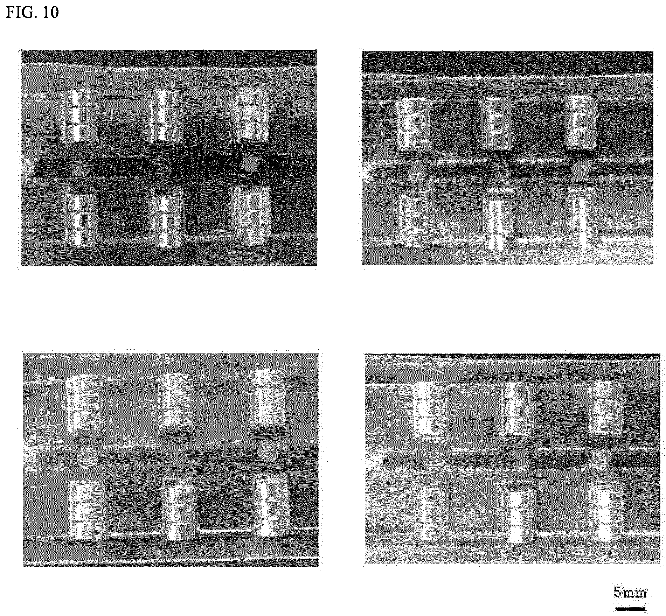

FIG. 10 is a diagram showing a culture appearance of organoid seeds produced according to Experimental Embodiment, which shows a process of injecting the organoid seeds into an organoid three-dimensional suspension culture apparatus using magnetic force and growing the organoid seeds while replacing the culture medium. It was confirmed that the organoid seeds maintained their shape and continued to grow despite periodic replacement of the culture medium.

## Claims

1. An organoid seed production method for organoid production, comprising:
a) forming organoid seeds suspended in a droplet state in a solution using an organoid seed composition in a microfluidic chip.

2. The organoid seed production method of claim 1, comprising:
b) gelling the organoid seeds in the droplet state.

3. The organoid seed production method of claim 1, wherein the organoid seed composition includes stem cells, growth factors, and biodegradable materials.

4. The organoid seed production method of claim 3, wherein the organoid seed composition includes an extracellular matrix.

5. The organoid seed production method of claim 3, wherein the organoid seed composition includes magnetic nanoparticles.

6. The organoid seed production method of claim 3, wherein the microfluidic chip includes a plate having a microchannel formed therein, and
the plate includes an insertion hole for inserting a syringe tip into the microchannel, a syringe tip extension region forming a front portion of the microchannel and through which the syringe tip extends, a first liquid port for injecting a solution into a first bonding part formed in the syringe tip extension region, and an outlet for collecting organoid seeds that move along the microchannel in a suspended state in the solution.

7. The organoid seed production method of claim 6, wherein the step a) includes
injecting the organoid seed composition into the microchannel at a first flow velocity V1 through the syringe tip; and
injecting the solution between the inner surface of the microchannel and the outer surface of the syringe tip at a second flow velocity V2,
wherein a relationship of V1 < V2 is formed.

8. The organoid seed production method of claim 7, wherein a surface tension N1 of the organoid seed composition and a surface tension N2 of the solution form a relationship of N1 > N2.

9. The organoid seed production method of claim 6, wherein the microfluidic chip includes a second bonding part formed on a path of the microchannel along which the organoid seeds move while being suspended in the solution, and a second liquid port for injecting a second solution into the second bonding part, and
the step b) is performed by injecting a gelation solution capable of gelling the organoid seeds in the droplet state into the second liquid port by reacting with the biodegradable material contained in the organoid seed composition.

10. The organoid seed production method of claim 6, wherein the biodegradable material of the organoid seed composition includes a biodegradable material that is gelled by UV, and
the step b) is performed by irradiating UV to the organoid seeds in the droplet state that move along the microchannel while being suspended in the solution.

11. An organoid seed production apparatus for producing organoids using an organoid seed composition, comprising:
a microfluidic chip formed of a plate including a microchannel; an insertion hole for inserting a syringe tip into the microchannel; a syringe tip extension region forming a part of the microchannel and through which the syringe tip extends; a first liquid port for injecting a solution into a first bonding part formed in the syringe tip region; and an outlet for collecting the organoid seeds that move along the microchannel in a suspended state in the solution;
a first syringe having the syringe tip at an end and containing the organoid seed composition therein and injecting the organoid seed composition into the microchannel through the syringe tip; and
a second syringe connected to the first liquid port, containing the solution and injecting the solution into the microchannel through the first bonding part.

12. The organoid seed production apparatus of claim 11, further comprising:
a first actuator connected to the first syringe to inject the organoid seed composition into the microchannel through the syringe tip; and
a second actuator connected to the second syringe to inject the solution into the microchannel,
wherein the first actuator injects the organoid seed composition into the microchannel at a first flow velocity V1 through the syringe tip,
the second actuator injects the solution between the inner surface of the microchannel and the outer surface of the syringe tip at a second flow velocity V2,
wherein a relationship of V1 < V2 is formed.

13. The organoid seed production apparatus of claim 12, wherein a surface tension N1 of the organoid seed composition and a surface tension N2 of the solution form a relationship of N1 > N2.

14. The organoid seed production apparatus of claim 11, wherein the microfluidic chip further includes a second bonding part formed on a path of the microchannel along which the organoid seeds move while being suspended in the solution, and a second liquid port for injecting a second solution into the second bonding part,
as the second solution, a gelation solution capable of gelling the organoid seed by reacting with a biodegradable material contained in the organoid seed composition is used to gel the organoid seeds.

15. The organoid seed production apparatus of claim 11, wherein the organoid seed composition includes a biodegradable material as a component so that the organoid seeds are gelled in response to UV, and
the organoid seed production apparatus comprises a UV irradiation device that irradiates UV to the organoid seeds in the droplet state moving along the microchannel.

16. The organoid seed production apparatus of claim 15, wherein the plate of the microfluidic chip includes a shielded seed formation region covered with a shielding cover and a UV irradiation region exposed to the UV irradiation device, and
UV is irradiated to the organoid seeds moving along the microchannel in the UV irradiation region.

17. The organoid seed production apparatus of claim 16, wherein in the microfluidic chip, an upper plate and a lower plate are bonded to form the plate,
the lower plate comprises a first lower plate corresponding to the shielded seed formation region and a second lower plate corresponding to the UV irradiation region, and
wherein the first lower plate is formed of a UV transparent material and the second lower plate is formed of a UV reflective material.
